# EUROPEAN PATENT APPLICATION

(11) **EP 2 100 611 A1**
(43) Date of publication of application: **16.09.2009**
(21) Application number: 07829850.2
(22) Date of filing: 16.10.2007
(51) Int. Cl.: A61K 31/44, A61P 3/04, A61P 3/10, C07D 213/75, C07D 265/30

(54) **GLUCOSE METABOLISM AMELIORATING AGENT**

(30) Priority: 18.10.2006 JP 2006283390
(71) Applicant: Shionogi&Co., Ltd., Osaka-shi, Osaka 5410045 (JP)
(72) Inventor: YUKIOKA, Hideo, Toyonaka-shi Osaka 5610825 (JP)
(74) Representative: Vossius & Partner
(86) International application number: PCT/JP2007/070116
(87) International publication number: WO 2008/047769

(57) **Abstract**

A compound of the formula I: 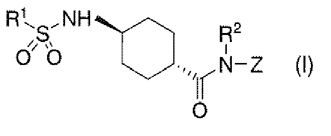 wherein
R¹ is lower alkyl,
R² is hydrogen or lower alkyl,
Z is optionally substituted lower alkyl, optionally substituted lower alkenyl, optionally substituted amino, optionally substituted lower alkoxy, optionally substituted carbocyclyl or optionally substituted heterocyclyl can stimulate the glucose metabolism independent of change in body weight. The compound can be used as a glucose metabolism stimulating agent.

## Description

### Field of the Invention

This invention relates to glucose metabolism stimulating agents.

### Background Art

Compounds of the formula I: 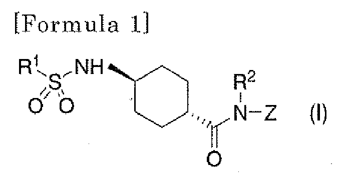 wherein
R¹ is lower alkyl,
R² is hydrogen or lower alkyl, and
Z is optionally substituted lower alkyl, optionally substituted lower alkenyl, optionally substituted amino, optionally substituted lower alkoxy, optionally substituted carbocyclyl or optionally substituted heterocyclyl,
are known for having NPY Y5 receptor specific antagonistic activity (Patent Document 1 or 2). However, there is no document to disclose or suggest that they can be used as a glucose metabolism stimulating agent.
[Patent Document 1] WO01/37826
[Patent Document 2] WO2006/001318

### Disclosure of Invention

### Problems to be solved by the Invention

The object of this invention is to provide glucose metabolism stimulating agents.

### Means for Solving the Problem

The inventors of this invention have intensively studied to find that plasma glucose levels in an obesity model mouse can be significantly and dose-dependently lowered by the compounds of the formula I: 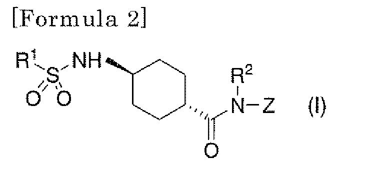 wherein
R¹ is lower alkyl,
R² is hydrogen or lower alkyl, and
Z is optionally substituted lower alkyl, optionally substituted lower alkenyl, optionally substituted amino, optionally substituted lower alkoxy, optionally substituted carbocyclyl or optionally substituted heterocyclyl. Therefore, the glucose metabolism stimulating agents of this invention are very useful as a therapeutic or preventive agent for diabetes.
The glucose metabolism stimulating agents of this invention can increase the glucose metabolism independent of changes in body weight. Therefore, the glucose metabolism stimulating agents of this invention can be useful not only for diabetic patients caused by obesity, but also for non-obese diabetic patients. The glucose metabolism stimulating agents of this invention have a distinct anti-diabetic action from existing anti-diabetic agents, and are useful for patients with diabetes resistant or inadequate response to existing anti-diabetic agents.

This invention includes the followings.
(1) A glucose metabolism stimulating agent comprising a compound of the formula I: 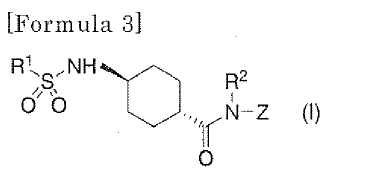 a pharmaceutically acceptable salt or solvate thereof as an active ingredient,
   wherein
   R¹ is lower alkyl,
   R² is hydrogen or lower alkyl, and
   Z is optionally substituted lower alkyl, optionally substituted lower alkenyl, optionally substituted amino, optionally substituted lower alkoxy, optionally substituted carbocyclyl or optionally substituted heterocyclyl.
(2) The glucose metabolism stimulating agent of (1), which is a therapeutic or preventive agent for diabetes.

Additionally, this invention also includes the followings.
(3) A method for stimulating the glucose metabolism characterized by administering a compound of the formula I: 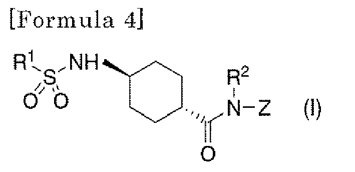 a pharmaceutically acceptable salt or solvate thereof,
   wherein
   R¹ is lower alkyl,
   R² is hydrogen or lower alkyl, and
   Z is optionally substituted lower alkyl, optionally substituted lower alkenyl, optionally substituted amino, optionally substituted lower alkoxy, optionally substituted carbocyclyl or optionally substituted heterocyclyl.
(4) Use of a compound of the formula I: 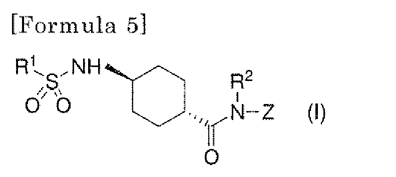 a pharmaceutically acceptable salt or solvate thereof,
   wherein
   R¹ is lower alkyl,
   R² is hydrogen or lower alkyl, and
   Z is optionally substituted lower alkyl, optionally substituted lower alkenyl, optionally substituted amino, optionally substituted lower alkoxy, optionally substituted carbocyclyl or optionally substituted heterocyclyl for the manufacture of a glucose metabolism stimulating agent.
(5) A method for the treatment or prevention of diabetes characterized by administering a compound of the formula I: 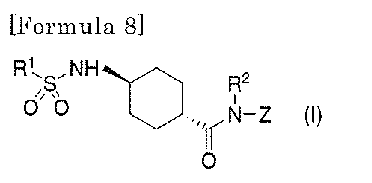 a pharmaceutically acceptable salt or solvate thereof,
   wherein
   R¹ is lower alkyl,
   R² is hydrogen or lower alkyl, and
   Z is optionally substituted lower alkyl, optionally substituted lower alkenyl, optionally substituted amino, optionally substituted lower alkoxy, optionally substituted carbocyclyl or optionally substituted heterocyclyl.
(6) Use of a compound of the formula I: 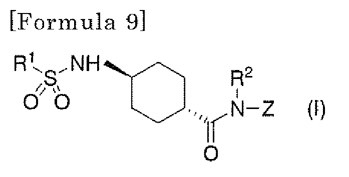 a pharmaceutically acceptable salt or solvate thereof,
wherein
R¹ is lower alkyl,
R² is hydrogen or lower alkyl, and
Z is optionally substituted lower alkyl, optionally substituted lower alkenyl, optionally substituted amino, optionally substituted lower alkoxy, optionally substituted carbocyclyl or optionally substituted heterocyclyl for the manufacture of a therapeutic or preventive agent for diabetes.

### Effect of the Invention

The glucose metabolism stimulating agents of this invention can be very useful as a therapeutic or preventive agent for diabetes. The glucose metabolism stimulating agents of this invention can be particularly useful not only for diabetic patients caused by obesity, but also for non-obese diabetic patients. The glucose metabolism stimulating agents of this invention have distinct mechanisms of action from the existing anti-diabetic agents and are useful for diabetic patients with no or inadequate response to existing anti-diabetic agents.

### Brief Description of Drawings

[Figure 1] Changes in plasma glucose levels in glucose tolerance test in mice (Administration of Compound (a) for 4 weeks)
[Figure 2] Body weight change in the glucose tolerance test in mice (Administration of Compound (a) for 4 weeks)
[Figure 3] Changes in plasma glucose levels in glucose tolerance test in mice (Short-term administration of Compound (a))
[Figure 4] Changes in plasma glucose levels in glucose tolerance test in mice (Administration of Compound (b) for 1 week)

### Best Mode for Carrying Out the Invention

The "glucose metabolism" is defined as follows. Sugar in food is digested into glucose, and the glucose is absorbed and then circulates throughout the body via blood flow. Liver and muscle tissues take in, and then utilize the glucose for energy production or store. Insulin, a hormone secreted by the pancreas, regulates glucose metabolism. Impaired glucose metabolism is a condition that abnormal high blood glucose levels to be hyperglycemia or low glucose levels to be hypoglycemia. The glucose metabolism stimulating agents of this invention can suppress plasma glucose levels and improve the hyperglycemia. The glucose metabolism stimulating agents of this invention can improve diabetes, complications of diabetes (e.g., diabetic neuropathy, diabetic nephropathy, diabetic retinopathy and the like), arteriosclerosis and the like.

A compound for the glucose metabolism stimulating agent of this invention is shown below.
A compound of the formula I: 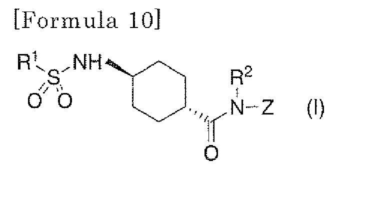 a pharmaceutically acceptable salt or solvate thereof,
wherein
R¹ is lower alkyl,
R² is hydrogen or lower alkyl,
Z is optionally substituted lower alkyl, optionally substituted lower alkenyl, optionally substituted amino, optionally substituted lower alkoxy, optionally substituted carbocyclyl or optionally substituted heterocyclyl.

The term "lower alkyl" of this description includes C1 to C10, preferably C1 to C6 and more preferably C1 to C3 straight or branched alkyl. Examples are methyl, ethyl, n-propyl, isopropyl, n-butyl, isobutyl, see-buthyl, tert-butyl, n-pentyl, isopentyl, neopentyl, hexyl, isohexyl, n-heptyl, isoheptyl, n-octyl, isooctyl, n-nonyl, n-decyl and the like. The lower alkyl represented by R¹ is preferably isopropyl or t-butyl.
Examples of substituent(s) of "optionally substituted lower alkyl" represented by Z are (1) halogen; (2) cyano;
(3) the following groups (i) to (xvi), which are optionally substituted with one or more substituent(s) selected from "Substituent group β" defined below,
(i) hydroxy, (ii) lower alkoxy, (iii) mercapto, (iv) lower alkylthio, (v) acyl, (vi) acyloxy, (vii) carboxy, (viii) lower alkoxycarbonyl, (ix) imino, (x) carbamoyl, (xi) thiocarbamoyl, (xii) lower alkylcarbamoyl, (xiii) lower alkylthiocarbamoyl, (xiv) amino, (xv) lower alkylamino, (xvi) heterocyclylcarbonyl and the like.

"Substituent group α" is a group constituting of (1) halogen; (2) oxo; (3) cyano; (4) nitro; (5) imino optionally substituted with lower alkyl or hydroxy; (6) the following groups (i) to (xxi), which are optionally substituted with one or more of group(s) selected from Substituent group β,
(i) hydroxy, (ii) lower alkyl, (iii) lower alkenyl, (iv) lower alkoxy, (v) carboxy, (vi) lower alkoxycarbonyl, (vii) acyl, (viii) acyloxy, (ix) imino, (x) mercapto, (xi) lower alkylthio, (xii) carbamoyl, (xiii) lower alkylcarbamoyl, (xiv) cycloalkylcarbamoyl, (xv) thiocarbamoyl, (xvi) lower alkylthiocarbamoyl, (xvii) lower alkylsulfinyl, (xviii) lower alkylsulfonyl, (xix) sulfamoyl, (xx) lower alkylsulfamoyl and (xxi) cycloalkylsulfamoyl;
(7) the following groups (i) to (v), which are optionally substituted with Substituent group β, lower alkyl, lower alkoxy(lower)alkyl, optionally protected hydroxy(lower)alkyl, halogeno(lower)alkyl, lower alkylsulfonyl and/or arylsulfonyl,
(i) cycloalkyl, (ii) cycloalkenyl, (iii)cycloalkyloxy, (iv) amino and (v) alkylenedioxy;
and
(8) the following groups (i) to (xii), which are optionally substituted with Substituent group β, lower alkyl, halogeno(lower)alkyl and/or oxo, (i) phenyl, (ii) naphthyl, (iii) phenoxy, (iv) phenyl(lower)alkoxy, (v) phenylthio, (vi) phenyl(lower)alkylthio, (vii) phenylazo, (viii) heterocyclyl, (ix) heterocyclyloxy, (x) heterocyclylthio, (xi) heterocyclylcarbonyl and (xii) heterocyclylsulfonyl.

"Substituent group β" is a group consisting of halogen, optionally protected hydroxy, mercapto, lower alkoxy, lower alkenyl, amino, lower alkylamino, lower alkoxycarbonylamino, lower alkylthio, acyl, carboxy, lower alkoxycarbonyl, carbamoyl, cyano, cycloalkyl, phenyl, phenoxy, lower alkylphenyl, lower alkoxyphenyl, halogenophenyl, naphthyl and heterocyclyl.

"Lower alkenyl" includes C2 to C10, preferably C2 to C8 and more preferably C3 to C6 straight or branched alkenyl having one or more double bonds at any possible positions. Examples are vinyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl and the like.
Examples of the substituent(s) for "optionally substituted lower alkenyl" are halogen, lower alkoxy, lower alkenyl, amino, lower alkylamino, lower alkoxycarbonylamino, lower alkylthio, acyl, carboxy, lower alkoxycarbonyl, carbamoyl, cyano, cycloalkyl, phenyl, lower alkylphenyl, lower alkoxyphenyl, naphthyl and/or heterocyclyl.

Examples of the substituent(s) for "optionally substituted amino" are the Substituent group β, optionally substituted benzoyl and/or optionally substituted heterocyclylcarbonyl wherein the substituent(s) are hydroxy, lower alkyl, lower alkoxy and/or lower alkylthio.

The lower alkyl part in "lower alkoxy", "lower alkylthio", "lower alkylcarbamoyl", "lower alkylthiocarbamoyl", "lower alkylamino,", "lower alkylsulfinyl", "lower alkylsulfonyl", "lower alkylsulfamoyl", "lower alkoxycarbonyl", "lower alkoxy(lower)alkyl", "hydroxyl(lower)alkyl", "lower alkoxycarbonylamino", "lower alkylphenyl", "lower alkoxyphenyl", "halogeno(lower)alkyl", "phenyl(lower)alkoxy" or "phenyl(lower)alkylthio" is the same as defined in the above "lower alkyl".

Examples of the substituent(s) for "optionally substituted lower alkoxy" are one or more substituent(s) selected from the above Substituent group β. Preferable examples are phenyl, lower alkylphenyl, lower alkoxyphenyl, naphthyl and heterocyclyl.
The term "acyl" includes (1) C1 to C10, preferably C1 to C6 and more preferably C1 to C4 straight or branched alkylcarbonyl or alkenylcarbonyl, (2) C4 to C9 and preferably C4 to C7 cycloalkylcarbonyl and (3) C7 to C11 arylcarbonyl. Examples are formyl, acetyl, propionyl, butyryl, isobutyryl, valeryl, pivaloyl, hexanoyl, acryloyl, propioloyl, methacryloyl, crotonoyl, cyclopropylcarbonyl, cyclohexylcarbonyl, cyclooctylcarbonyl, benzoyl and the like.
The "acyl" part in "acyloxy" is the same as the above.
The term "protective group" in "optionally protected hydroxyl" and "optionally protected hydroxy lower alkyl" includes all of hydroxy protecting groups usually used. For example, acyl such as acetyl, trichloroacetyl and benzoyl and the like, lower alkoxycarbonyl such as t-butoxycarbonyl and the like, lower alkylsulfonyl such as methane sulfonyl and the like, lower alkoxy(lower)alkyl such as methoxymethyl and the like and trialkylsilyl such as t-butyldimethylsilyl and the like are included.
The term "halogen" includes fluorine, chlorine, bromine and iodine.
Especially, fluorine or chlorine is preferable.
The halogen part in "halogenophenyl" and "halogeno(lower)alkyl" is the same as the above "halogen".
The term "lower alkylene" includes a bivalent group comprising 1 to 6 of methylene, preferably 2 to 6 of methylene and more preferably 3 to 6 of methylene. For example, methylene, ethylene, trimethylene, tetramethylene, pentamethylene, hexamethylene and the like are included. Tetramethylene is especially preferable.
The lower alkylene part in "alkylenedioxy" is the same as the above "lower alkylene". Methylenedioxy or ethylenedioxy is preferable.

The term "carbocyclyl" includes the above "cycloalkyl", "cycloalkenyl", "bicycloalkyl" and "aryl".
The term "cycloalkyl," includes C3 to C8 and preferably C5 to C6 cyclic alkyl. Examples are cyclopropyl, cyclobutyl, cyclopentyl, cyclohexyl, cycloheptyl, cyclooctyl and the like.
The term "cycloalkenyl" includes a group having at least one double bond at any possible positions in the above cycloalkyl. Examples are cyclopropenyl, cyclobutenyl, cyclopentenyl, cyclohexenyl, cyclohexadienyl and the like.
The term "bicycloalkyl" includes a group which is formed by excluding one hydrogen atom from a C5 to C8 aliphatic cycle containing two rings which possess two or more of atoms in common. Examples are bicyclo[2.1.0]pentyl, bicyclo[2.2.1]heptyl, bicyclo[2.2.2]octyl, bicyclo[3.2.1]octyl and the like.
The term "aryl" includes a monocyclic or polycyclic aromatic carbocyclyl group. Examples are phenyl, naphthyl, anthryl, phenanthryl and the like. "Aryl" includes aryl fused with other a non-aromatic carbocyclyl group. Examples are indanyl, indenyl, biphenylyl, acenaphthyl, tetrahydronaphthyl, fluorenyl and the like. Phenyl is especially preferable.
Examples of the substituent(s) of "optionally substituted carbocyclyl" include one or more substituent(s) selected from the above Substituent group α or Substituent group B. The carbocyclyl may be optionally substituted at any position.

The cycloalkyl part in "cycloalkylcarbamoyl", "cycloalkylsulfamoyl" and "cycloalkyloxy" is the same as the above "cycloalkyl.".
The aryl part in "arylsulfonyl" is the same as the above "aryl".

The term ``heterocyclyl" includes a heterocyclic group containing at least one heteroatom arbitrarily selected from O, S and N. For example, 5- or 6-membered heteroaryl such as pyrrolyl, imidazolyl, pyrazolyl, pyridyl, pyridazinyl, pyrimidinyl, pyrazinyl, triazolyl, triazinyl, tetrazolyl, isoxazolyl, oxazolyl, oxadiazolyl, isothiazolyl, thiazolyl, thiadiazolyl, furyl, thienyl and the like; fused heterocyclyl consisting of two rings such as indolyl, isoindolyl, indazolyl, indolizinyl, indolinyl, isoindolinyl, quinolyl, isoquinolyl, cinnolinyl, phthalazinyl, quinazolinyl, naphthyridinyl, quinoxalinyl, purinyl, pteridinyl, benzopyranyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazoropyridyl, imidazothiazolyl, pyrazinopyridazinyl, quinazolinyl, naphthyridinyl, dihydropyridyl, tetrahydroquinolyl, tetrahydrobenzothienyl and the like; fused heterocyclyl consisting of three rings such as carbazolyl, acridinyl, xanthenyl, phenothiazinyl, phenoxathiinyl, phenoxazinyl, dibenzofuryl and the like; and non-aromatic heterocyclyl such as dioxanyl, thiiranyl, oxiranyl, oxathiolanyl, azetidinyl, thianyl, pyrrolidinyl, pyrrolinyl, imidazolidinyl, imidazolinyl, pyrazolidinyl, pyrazolinyl, piperidyl, piperazinyl, morpholinyl, morpholino, thiomorpholinyl, thiomorpholino, dihydropyridyl, tetrahydrofuryl, tetrahydropyranyl, tetrahydrothiazolyl, tetrahydroisothiazolyl and the like.
"Fused heterocyclyl" fused with a ring other than a heterocycle (e.g., benzothiazolyl and the like), may connect at any possible position.
Preferable examples of "heterocyclyl" represented by Z includes imidazolyl, benzothiazolyl, isothiazolyl., benzopyranyl, morpholino, pyridyl, quinolyl, pyrimidyl and the like.
Examples of the substituent(s) of "optionally substituted heterocyclyl" are the same as the substituent(s) of the above "carbocyclyl".
Heterocyclyl parts in "heterocyclyloxy", "heterocyclylthio", "heterocyclylcarbonyl" and "heterocyclylsulfonyl" are the same as the above "heterocyclyl".

Examples of "the pharmaceutically acceptable salt" are salts witch mineral acids such as hydrochloric acid, sulfuric acid, nitric acid, phosphoric acid and the like; salts with organic acids such as para-toluenesulfonic acid, methanesulfonic acid, oxalic acid, citric acid and the like; salts with organic bases such as ammonium, trimethylammonium, triethylammonium and the like; salts with alkaline metals such as sodium, potassium and the like; and salts with alkaline earth metals such as calcium, magnesium and the like.

"Solvate" means that a compound of the present invention can be coordinate any number of solvent molecules to Compound (I). Hydrate is preferable.

When Compound (I) of the present invention has an asymmetric carbon atom, it contained racemic body and all stereoisomers (a diastereoisomer, an antipode or the like). When Compound (I) of the present invention has a double bond and there is geometrical isomer at a substituent position of the double bond, it includes both type of the isomers.

As Compound (I) used for the glucose metabolism stimulating agent of this invention, the preferable examples are the following compounds, their pharmaceutically acceptable salts or solvates thereof.

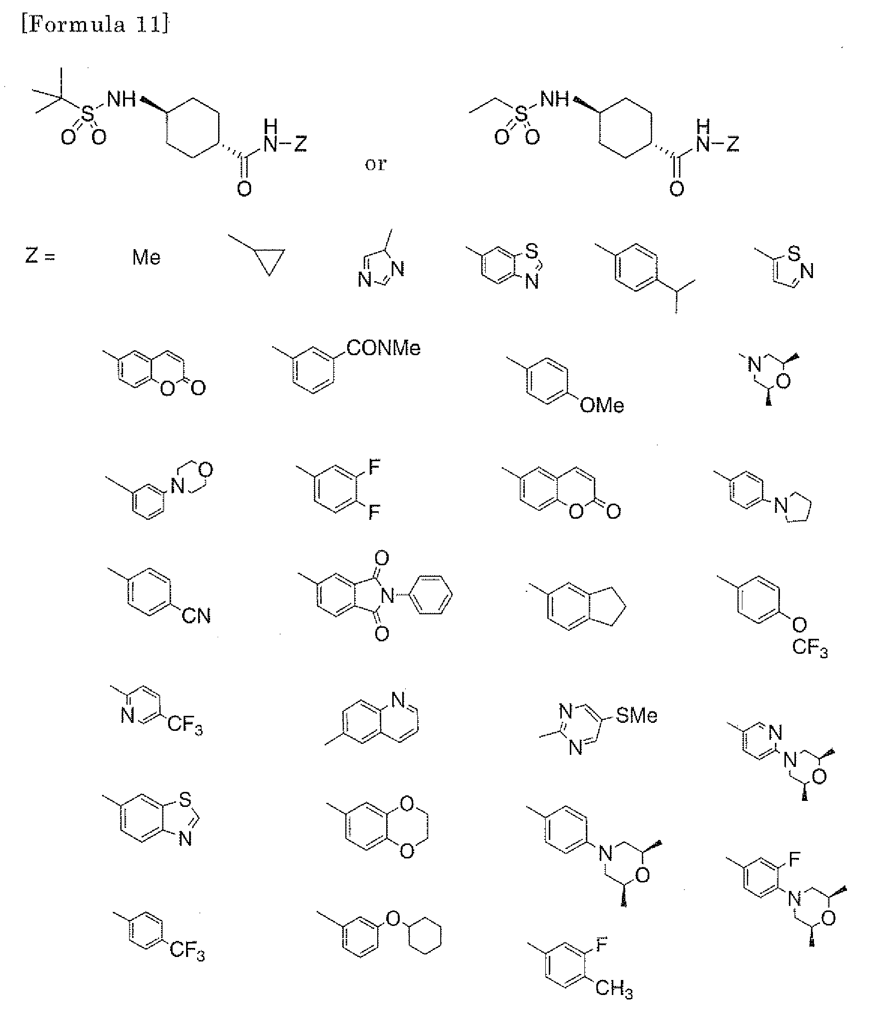

The more preferable examples are the following compounds.

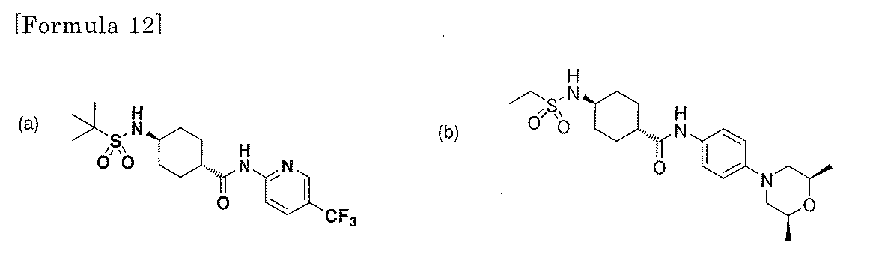

Compound (I) used for the glucose metabolism stimulating agent of this invention can be prepared by the method described in WO01/37826, WO2003/076374, WO2006/001318 or JP2005-255630.

To be more precise, the following methods are exemplified. 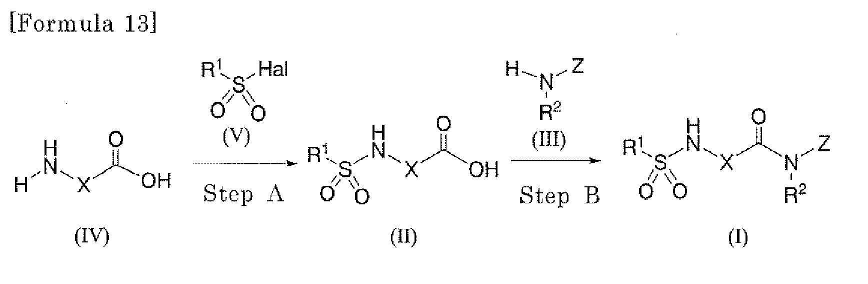 wherein Hal is halogen, X is cyclohexylene, and the other symbols are the same as the above.

### Step A

Compound (IV) is reacted with Compound (V) having the desired substituent R¹ in a suitable solvent, optionally in the presence of a base to give Compound (II).
Examples of the solvent are tetrahydrofuran, dimethylformamide, diethyl ether, dichloromethane, toluene, benzene, xylene, cyclohexane, hexane, chloroform, ethyl acetate, butyl acetate, pentane, heptane, dioxane, acetone, acetonitrile, water and a mixture thereof. Dioxane, dichloromethane and the like are preferable.
Examples of the base include sodium hydroxide, potassium hydroxide and lithium hydroxide.
The reaction temperature is about 0 °C to 50 °C and preferably about 20 to 30 °C.
The reaction time is about 5 minutes to 30 hours and preferably about 5 hours to 20 hours.
As Compound (IV) and Compound (V), a known compound or a compound synthesized from a known compound by a known method can be used.

### Step B

Compound (II) is reacted with Compound (III) having the desired substituent Z and R² in a suitable solvent.
Examples of the solvent are tetrahydrofuran, dimethylformamide, diethyl ether, dichloromethane, toluene, benzene, xylene, cyclohexane, hexane, chloroform, ethyl acetate, butyl acetate, pentane, heptane, dioxane, acetone, acetonitrile, water and a mixture thereof. Dimetylformamide, tetrahydrofuran, ethyl acetate and the like are preferable.
If necessary, the reaction can be carried out under the presence of a condensing agent(s) such as 1,3-dicyclohexyl carbodiimide, 1-ethyl-3-(3-dimethylamino) carbodiimide (WSCD; water-soluble carbodiimide) and the like and/or acidic additives such as 1-hydroxybenzotriazole, 3,4-dihydro-3-hydroxy-4-oxo -1,2,3-benzotriazine and the like.
The reaction temperature is about 0 °C to 50 °C and preferably about 20 °C to 30 °C.
The reaction time is about 5 minutes to 30 hours and preferably about 5 hours to 20 hours.

Amino groups may be protected with a suitable protecting group in the usual manner at a suitable step. Examples of the protecting group includes phthalimide, lower alkoxycarbonyl, lower alkenyloxycarbonyl, halogenoalkoxycarbonyl, aryl(lower)alkoxycarbonyl, trialkylsilyl, lower alkylsulfonyl, halogeno(lower)alkylsulfonyl, arylsulfonyl, lower alkylcarbonyl and arylcarbonyl.
After protection of the amino group, the compound is subjected to the above-mentioned reactions and the obtained compound is deprotected by treatment of an acid or base in a suitable solvent at a suitable stage. Examples of the solvent include tetrahydrofuran, dimethylformamide, diethyl ether, dichloromethane, toluene, benzene, xylene, cyclohexane, hexane, chloroform, ethyl acetate, butyl acetate, pentane, heptane, dioxane, acetone, acetonitrile and the mixture thereof. Examples of the base include hydrazine, pyridine, sodium hydroxide and potassium hydroxide. Examples of the acid include hydrochloric acid, trifluoroacetic acid and hydrofluoric acid.

The glucose metabolism stimulating agents of this invention can be administered orally or parenterally. In the case of oral administration, they may be in any usual form such as tablets, granules, powders, capsules, pills, solutions, syrups, buccal tablets and sublingual tablets. When the compounds are parenterally administered, any usual form is preferable, for example, injections (e.g., intravenous, intramuscular), suppositories, endermic agents and inhalations. Oral administration is especially preferable because the compounds of the present invention show a high oral absorbability.

A pharmaceutical composition may be manufactured by mixing an effective amount of Compound (I) used for the glucose metabolism stimulating agent of this invention with various pharmaceutical additives suitable for the administration form, such as excipients, binders, moistening agents, disintegrants, lubricants and diluents. For an injection, an active ingredient together with a suitable carrier can be sterilized to give a pharmaceutical composition.

Examples of the excipients include lactose, saccharose, glucose, starch, calcium carbonate and crystalline cellulose. Examples of the binders include methylcellulose, carboxymethylcellulose, hydroxypropylcellulose, gelatin and polyvinylpyrrolidone. Examples of the disintegrants include carboxymethylcellulose, sodium carboxymethylcellulose, starch, sodium alginate, agar and sodium lauryl sulfate. Examples of the lubricants include talc, magnesium stearate and macrogol. Cacao oil, macrogol, methylcellulose or the like may be used as base materials of suppositories. When the composition is manufactured as solutions, emulsified injections or suspended injections, solubilizing agents, suspending agents, emulsifiers, stabilizers, preservatives, isotonic agents and the like which are usually used may be added. For oral administration, sweetening agents, flavors and the like which are usually used may be added.

Although the dosage of the glucose metabolism stimulating agents of this invention should be determined in consideration of the patient's age and body weight, the type and severity of diseases, the administration route and the like, a usual oral dosage for an adult is 0.05 to 100 mg/kg/day and preferable is 0.1 to 10 mg/kg/day of an active ingredient, Compound (I) used for the glucose metabolism stimulating agent of this invention. For parenteral administration, although the dosage highly varies with administration routes, a usual dosage is 0.005 to 10 mg/kg/day and preferably 0.01 to 1 mg/kg/day of an active ingredient, Compound (I) used for the glucose metabolism stimulating agent of this invention. The dosage may be administered in one to several divisions per day.

The present invention is further explained by the following Examples, which are not intended to limit the scope of the present invention.

The following Examples 1 to 3 were carried out in an obesity model mouse, male C57BL/6J-ob/ob mice (12-17 week old, 40-60g) to study the effect of Compound (a) on glucose metabolism in mice.

### Example 1

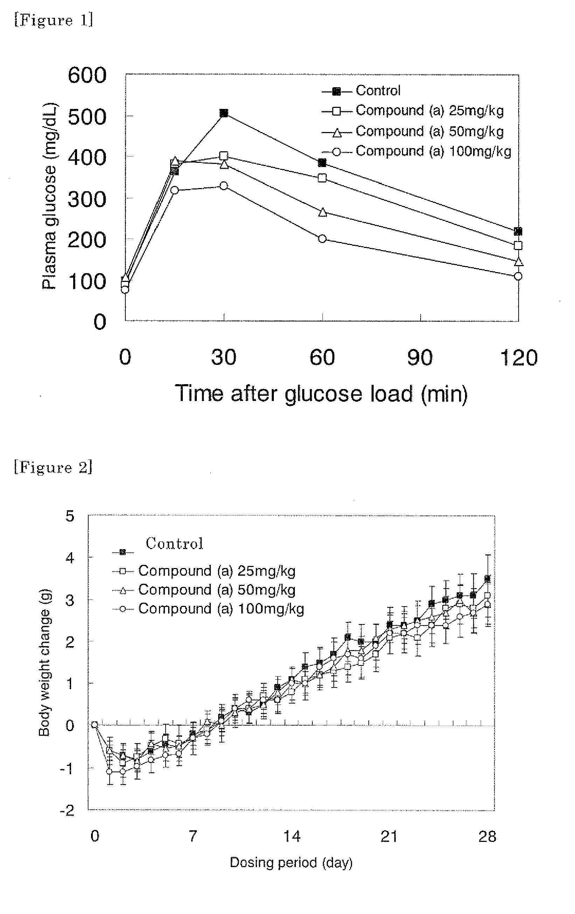

Hydroxypropylmethyl cellulose solution (0.5%w/v, Shin-Etsu Chemical Co., Ltd) or Compound (a) or (b) suspended in the solution were orally administered at 25, 50 or 100 mg/kg twice a day (8:00-10:00, 16:30-18:00) for 4 weeks. Food was removed in the morning (8-00-9:00) of the last day of administration. Mice were kept under fasting until the end of glucose tolerance test. In this period, the mice were allowed free access to water. The next day, glucose tolerance test was carried out in the afternoon (13:00-16:00). Blood was collected from tail veins just before and at 15, 30, 60 and 120 minutes after oral administration of glucose (2 g/kg) to examine plasma glucose levels. AUC (area under the curve) was calculated by the trapezoidal method from the graphs made with obtained values (Figure 1). AUCs in mice treated with Compound (a) at 25 mg/kg, 50 mg/kg and 100mg/kg were 25610 mg·min/dL, 20315 mg·min/dL and 15848 mg·min/dL, respectively. AUC in control mice (dosed with 0.5 % hydroxypropylmethyl cellulose solution) was 29863 mg·min/dL. These results showed that AUC in mice dosed with Compound (a) decreased with dose-dependent manner. The AUC in mice dosed with Compound (a) at 100 mg/kg was a statistically significant decrease compared to that seen in control mice.
There is no statistically significant change in body weight during the dosing period (Figure 2).

### Example 2

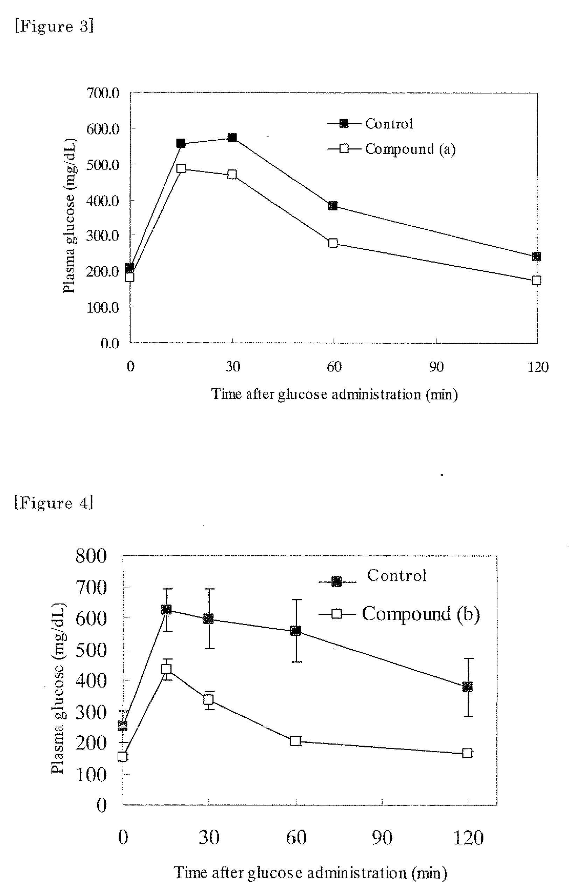

Compound (a) was administered to mice for short term and then glucose tolerance test was performed. In the morning (8:00-10:00) of the previous day of the test, hydroxypropylmethyl cellulose solution (0.5%w/v, Shin-Etsu Chemical Co., Ltd) or Compound (a) at 100 mg/kg suspended in the solution was administered by oral gavage. Food was removed just after the administration. Mice were kept under fasting until the end of glucose tolerance test. In this period, the mice were allowed free access to water. The oral administration was also performed in the evening of the previous day of the test (17:00-18:00) and in the morning of the day of the test (8:00-9:00). Glucose tolerance test was carried out in the afternoon (13:00-16:00), and AUC was calculated as described in Example 1 (Figure 3). AUC in mice dosed with Compound (a) was 22423 mg· min/dL, on the other hand, AUC in control mice (dosed with 0.5 % hydroxypropylmethyl cellulose solution) was 15033 mg·min/dL. The AUC in mice dosed with Compound (a) was a statistically significant decrease compared to that seen in control mice.

### Example 3

Hydroxypropylmethyl cellulose solution (0.5 %w/v, Shin-Etsu Chemical Co., Ltd) or Compound (b) suspended in the solution were orally administered at 100 mg/kg twice a day (8:00-10:00, 16:30-18:00) for a week. Food was removed in the morning (8:00-9:00) of the last day of administration. Mice were kept under fasting until the end of glucose tolerance test. In this period, the mice were allowed free access to water. The next day, glucose tolerance test was carried out in the afternoon (13:00-16:00) and then AUC was calculated as described in Example 1 (Figure 4). AUC in mice dosed with Compound (b) was 15546 mg·min/dL, on the other hand, AUC in control mice (dosed with 0.5 % hydroxypropylmethyl cellulose solution) was 22532 mg·min/dL. The AUC in mice dosed with Compound (b) was a statistically significant decrease compared to that seen in control mice.

### Industrial Applicability

The glucose metabolism stimulating agents of this invention are very useful as a therapeutic or preventive agent for diabetes. The glucose metabolism stimulating agents of this invention can be particularly useful not only for diabetic patients caused by obesity, but also for non-obese diabetic patients. Therefore, the glucose metabolism stimulating agents of this invention have distinct mechanisms of action from existing anti-diabetic agents and are useful for diabetic patients with no or inadequate response to existing anti-diabetic agents.

## Claims

1. A glucose metabolism stimulating agent comprising a compound of the formula I: 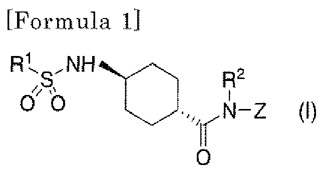 a pharmaceutically acceptable salt or solvate thereof as an active ingredient,
wherein
R¹ is lower alkyl,
R² is hydrogen or lower alkyl,
Z is optionally substituted lower alkyl, optionally substituted lower alkenyl, optionally substituted amino, optionally substituted lower alkoxy, optionally substituted carbocyclyl or optionally substituted heterocyclyl.

2. The glucose metabolism stimulating agent of Claim 1, which is a therapeutic or preventive agent for diabetes.

3. A method for stimulating the glucose metabolism **characterized by** administering a compound of the formula I: 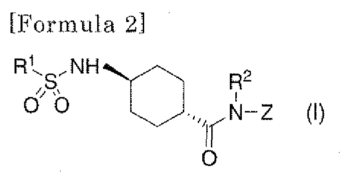 a pharmaceutically acceptable salt or solvate thereof,
wherein
R¹ is lower alkyl,
R² is hydrogen or lower alkyl, and
Z is optionally substituted lower alkyl, optionally substituted lower alkenyl, optionally substituted amino, optionally substituted lower alkoxy, optionally substitute carbocyclyl or optionally substitute heterocyclyl.

4. Use of a compound of the formula I: 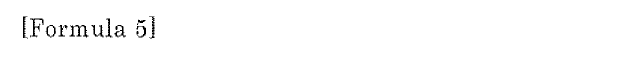 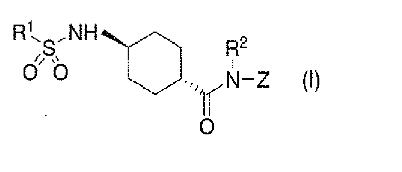 a pharmaceutically acceptable salt or solvate thereof,
wherein
R¹ is lower alkyl,
R² is hydrogen or lower alkyl, and
Z is optionally substituted lower alkyl, optionally substituted lower alkenyl, optionally substituted amino, optionally substituted lower alkoxy, optionally substituted carbocyclyl or optionally substituted heterocyclyl
for the manufacture of a glucose metabolism stimulating agent.

5. A method for the treatment or prevention of diabetes **characterized by** administering a compound of the formula I: 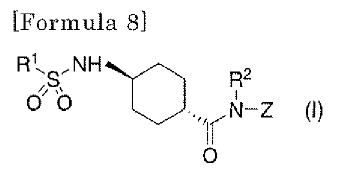 a pharmaceutically acceptable salt or solvate thereof,
wherein
R¹ is lower alkyl,
R² is hydrogen or lower alkyl, and
Z is optionally substituted lower alkyl, optionally substituted lower alkenyl, optionally substituted amino, optionally substituted lower alkoxy, optionally substituted carbocyclyl or optionally substituted heterocyclyl.

6. Use of a compound of the formula I: 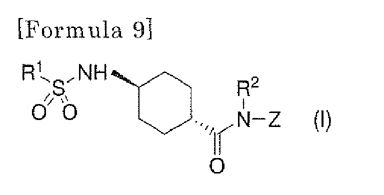 a pharmaceutically acceptable salt or solvate thereof,
wherein
R¹ is lower alkyl,
R² is hydrogen or lower alkyl, and
Z is optionally substituted lower alkyl, optionally substituted lower alkenyl, optionally substituted amino, optionally substituted lower alkoxy, optionally substituted carbocyclyl or optionally substituted heterocyclyl
for the manufacture of a therapeutic or preventive agent for diabetes.
